# EUROPÄISCHE PATENTANMELDUNG

(11) **EP 1 325 902 A1**
(43) Veröffentlichungstag der Anmeldung: **09.07.2003**
(21) Anmeldenummer: 02028773.6
(22) Anmeldetag: 23.12.2002
(51) Int. Cl.: C07C 51/44, C07C 51/46, C07C 51/43, C07C 51/09, C07C 57/10

(54) **Verfahren zur Herstellung von Sorbinsäure**

(30) Priorität: 04.01.2002 DE 10200200
(71) Anmelder: Nutrinova Nutrition Specialties & Food Ingredients GmbH, 65929 Frankfurt am Main (DE)
(72) Erfinder: Mollenkopf, Christoph, Dr., 65929 Frankfurt am Main (DE)
(74) Vertreter: Schweitzer, Klaus, Dr.

(57) **Zusammenfassung**

Ein Verfahren zur Herstellung von Sorbinsäure durch thermische Spaltung des aus Crotonaldehyd und Keten hergestellten Polyesters in Gegenwart eines Lösungsmittels und eines Amins als Katalysator unter gleichzeitigem Abdestillieren der gebildeten Sorbinsäure und des Lösungsmittels über eine Rektifikationskolonne mit Rücklauf, das dadurch gekennzeichnet ist, dass als Rücklauf nicht das Destillat sondern nur das Lösungsmittel verwendet wird, führt zu hohen Ausbeuten an Sorbinsäure und zu einem verringerten Verbrauch an Katalysatoramin.

## Beschreibung

Die vorliegende Erfindung betrifft ein Verfahren zur Herstellung von Sorbinsäure.

Zur Herstellung von Sorbinsäure sind verschiedene Verfahren bekannt.

Ein besonders wirtschaftliches Verfahren geht von dem polymeren Reaktionsprodukt Polyester aus, der durch Umsetzung von Crotonaldehyd mit Keten in einem inerten Lösungsmittel in Gegenwart eines fettsauren Salzes eines zwei- und/oder dreiwertigen Metalls der II. bis VIII. Nebengruppe des Periodensystems als Katalysator hergestellt wird. Die Sorbinsäure wird daraus durch alkalische Verseifung und Reaktion mit einer starken Säure gewonnen (DE-AS 1 042 573).

Aus diesem Polyester kann jedoch auf verschiedenen Wegen Sorbinsäure gewonnen werden.

In der DE-AS 1 059 899 wird die Spaltung des Polyesters zu Sorbinsäure in Gegenwart eines inerten oberhalb von 150 °C siedenden Lösungsmittels beschrieben. Das Lösungsmittel dient gleichzeitig als Lösungsmittel für den Polyester und als Schleppmittel zur Destillation der daraus gebildeten Sorbinsäure. Als Katalysator werden dem Polyester bzw. dem diesen enthaltenden Gemisch Alkalihydroxide oder alkalisch reagierende Salze organischer Säuren zugesetzt.

Ein technisch bedeutsames Verfahren zur Sorbinsäureherstellung besteht darin, dass man die Spaltung des Polyesters in Gegenwart eines oberhalb 150 °C siedenden Amins als Katalysator bei Temperaturen von 160 bis 220 °C unter gleichzeitigem Abdestillieren der Sorbinsäure und des Lösungsmittels durchführt (DE-AS 1 282 645). Als Lösungsmittel werden dabei die in der DE-AS 1 059 899 genannten Lösungsmittel, insbesondere die dort genannten aliphatischen Carbonsäuren mit entsprechendem Siedepunkt, verwendet.

Bei der technischen Durchführung dieses Verfahrens wird die Polyesterspaltung in einer kontinuierlich betriebenen Destillationsapparatur durchgeführt. Der in dem Lösungsmittel gelöste Sorbinsäurepolyester wird in die Destillationsblase eindosiert, wo die aminkatalysierte Spaltung des Sorbinsäurepolyesters zur Sorbinsäure stattfindet. Die gebildete Sorbinsäure wird zusammen mit dem Lösungsmittel über eine Rektifikationskolonne mit Rücklauf abdestilliert. Der Rücklauf ist somit das Destillat, d.h. eine Mischung aus Lösungsmittel und Sorbinsäure. Die Rektifikation ist notwendig, um den Übergang des Amins in das Destillat zu verhindern und um eine entsprechende Reinheit der Sorbinsäure zu erzielen. Aus dem Destillat wird anschließend die Sorbinsäure auskristallisiert und von dem Lösungsmittel abgetrennt. Das Lösungsmittel wird im Kreis geführt.

Bei der thermischen Sorbinsäurepolyesterspaltung entstehen in der Destillationsblase neben der Sorbinsäure auch Polymere, die kontinuierlich ausgeschleust werden müssen. Dabei wird jedoch auch Katalysatoramin mit ausgeschleust. Zum einen bedeutet dies einen nicht unerheblichen Ausbeuteverlust durch die Polymerbildung, zum anderen muss Katalysatoramin kontinuierlich zugegeben werden, um die Verluste durch die Ausschleusung auszugleichen.

Es bestand daher die Aufgabe, ein Verfahren zu entwickeln, bei dem während der Sorbinsäurepolyesterspaltung die Polymerbildung verringert und damit sowohl die Ausbeute an Sorbinsäure erhöht als auch der Verbrauch an Amin verringert wird.

Überraschenderweise wurde nun gefunden, dass die Polymerisatbildung während der Destillation deutlich verringert werden kann, wenn als Rücklauf nicht wie im Stand der Technik das Destillat sondern lediglich das Lösungsmittel verwendet wird. Das Ergebnis ist eine sehr reine Sorbinsäure bei gleichzeitiger Ausbeuteerhöhung und einer Verminderung des notwendigen Einsatzes von Amin als Katalysator.

Gegenstand der vorliegenden Erfindung ist somit ein Verfahren zur Herstellung von Sorbinsäure durch thermische Spaltung des aus Crotonaldehyd und Keten hergestellten Polyesters in Gegenwart eines Lösungsmittels und eines Amins als Katalysator unter gleichzeitigem Abdestillieren der Sorbinsäure und des Lösungsmittels über eine Rektifikationskolonne mit Rücklauf, dadurch gekennzeichnet, dass als Rücklauf das Lösungsmittel verwendet wird.

Vorteilhaft lässt sich das erfindungsgemäße Verfahren so durchführen, dass die gesamten anfallenden Brüden zur Kristallisation der Sorbinsäure abgekühlt werden, die auskristallisierte Sorbinsäure abgetrennt wird und ein Teil des Lösungsmittels wieder auf Kopftemperatur erwärmt und als Rücklauf über den Kolonnenkopf in die Rektifikationskolonne eingespeist wird. Das verbleibende Lösungsmittel wird, wie ursprünglich auch, zum Verdünnen des zum Einsatz kommenden Sorbinsäurepolyesters verwendet. Im Unterschied zum Stand der Technik, bei dem der für die Rektifikation notwendige Rücklauf aus Destillat besteht, wird bei dem erfindungsgemäßen Verfahren das Lösungsmittel als Rücklauf verwendet

Zur Erreichung des erfindungsgemäßen Zwecks ist es vorteilhaft, wenn das als Rücklauf verwendete Lösungsmittel möglichst vollständig von der im Destillat enthaltenen Sorbinsäure befreit worden ist, dies ist jedoch nicht zwingend erforderlich. Mit Zunahme des Sorbinsäureanteils am Rücklauf werden allerdings die mit dem erfindungsgemäßen Verfahren gegenüber dem Stand der Technik zu erzielenden Vorteile geringer. Diese weniger optimalen Ausführungsformen werden jedoch von der Erfindung mit umfasst.

Das Fließschema 1 zeigt schematisch das gemäß dem Stand der Technik bei der Polyesterspaltung benutzte Destillationsverfahren, bei dem am Kolonnenkopf abgenommenes Destillat (Gemisch aus Sorbinsäure und Lösungsmittel) als Rücklauf in die Rektifikationskolonne eingespeist wird.

Fließschema 2 zeigt schematisch das Destillationsverfahren gemäß der vorliegenden Erfindung, bei dem das abdestillierte Lösungsmittel nach Abtrennung der Sorbinsäure als Rücklauf in die Rektifikationskolonne eingespeist wird.

Außer dem Effekt der Ausbeuteerhöhung erlaubt die erfindungsgemäße Fahrweise auch eine bis zu 30 %ige Kapazitätssteigerung, allerdings unter zumindest teilweisem Verlust des Ausbeutegewinns. Zur Erzielung der Kapazitätserhöhung bei der oben beschriebenen erfindungsgemäßen Fahrweise mit Lösungsmittel als Rücklauf wird ein Teil des Lösungsmittels im Einsatz durch Sorbinsäurepolyester ersetzt, d. h. gemessen am Polyestereinsatz in der Destillationsblase wird weniger Lösungsmittel verwendet. Erst bei einer größer als 30 %igen Kapazitätssteigerung sinkt die Ausbeute an Sorbinsäure wieder unter die Ausbeute, die bei der normalen, dem Stand der Technik entsprechenden Fahrweise erzielbar ist.

Die erfindungsgemäße Anordnung und Fahrweise zur Herstellung von Sorbinsäure erlaubt also eine große Flexibilität bzgl. Produktionsmenge und Anlagenauslastung, mit der sehr schnell auf Markterfordernisse reagiert werden kann.

Grundsätzlich kann das erfindungsgemäße Verfahren zur Sorbinsäureherstellung wie in der DE-AS 1 282 645 beschrieben durchgeführt werden, ohne allerdings darauf beschränkt zu sein. Insoweit wird ausdrücklich auf diese Schrift bezug genommen und die Beschreibung der Sorbinsäureherstellung unter Verwendung katalytischer Mengen eines Amins (bevorzugt 0,5 bis 10 Gewichtsprozent bezogen auf den eingesetzten Polyester) als Teil der Beschreibung der vorliegenden Erfindung aufgenommen (vgl. dazu die Offenbarung ab Spalte 3, Zeile 31 mit Ausnahme der Abschnitte, die die Verwendung eines hohen Aminüberschusses beschreiben, wobei das Amin gleichzeitig Katalysator und Schleppmittel für die Sorbinsäure (-destillation) ist). Diese Bezugnahme gilt auch für die dort beschriebenen bevorzugten Lösungsmittel, Amine, Gewichts- und/oder Mengenverhältnisse der Ausgangsstoffe und Reaktions- bzw. Destillationstemperaturen.

Insbesondere wird im folgenden auf zusätzliche besonders vorteilhafte Ausgestaltungen des erfindungsgemäßen Verfahrens hingewiesen.

Gemäß DE-AS 1 282 645 sind sekundäre oder tertiäre aliphatische, alicyclische, 5-oder 6-gliedrige heterocyclische Stickstoff und/oder Sauerstoff enthaltende oder aliphatisch-aromatisch substituierte, unter Normaldruck oberhalb 100 °C siedende Amine als Katalysatoren geeignet.

Mit gutem Erfolg lassen sich zum Beispiel: Methyloctadecylamin, Dimethyloctadecylamin, Dimethylhexadecylamin, Dimethyltetradecylamin, Dimethyldodecylamin, Dibutyldodecylamin, N,N,N',N'-Tretramethylhexamethylendiamin, N,N,N'-Trimethyl-N'-phenylethylendiamin, N-Octadecylpyrrolidon, N-Octadecylpiperidin, N-Dodecylmorpholin, N,N'-Dipropylpiperazin, α-Hexylpyrrolidon, Triethylentetramin, Ethyl-bis-(β-ethylaminoethyl)-amin, 1-Octyldiethylentriamin, Ethylenglycol-bis-(2-methylaminoethylether), Dioctadecylamin, Diethylentriamin, Trioctadecylamin, Trioctylamin, Tricyclohexylamin, Dimethylstearylamin einsetzen.

Zur Gewinnung der Sorbinsäure sind als Lösungsmittel aliphatische, alicyclische, aromatische Kohlenwasserstoffe, deren Chlor-, Brom- und Nitroderivate sowie auch Ether und Siliconöle geeignet, deren Siedepunkt bei normalem Druck bevorzugt über 150 °C, vorzugsweise über 180 °C liegt. Aber auch Ketone, Ester, Carbonsäuren und Alkohole mit dem entsprechenden Siedebereich können als Lösungsmittel herangezogen werden, obwohl im allgemeinen die Ergebnisse nicht ganz so gut sind, da sie sich vermutlich teilweise mit dem Reaktionsgemisch umsetzen. Es ist zweckmäßig, solche Lösungmittel zu verwenden, die bei normalen Temperaturen flüssig sind, bei normalem Druck unter 300 °C, vorzugsweise unter 270 °C sieden und mit der Sorbinsäure azeotrope Gemische bilden, so dass sie auch gleichzeitig als Träger- oder Schleppmittel dienen. Beispiele sind Petroleumfraktionen, Dodekan, Tetradekan, 5-Methyldodekan, Dodecen, Dicyclohexylmethan, p-Di-tertiär-butylbenzol, 1-Methylnaphthalin, 2-Methylnaphthalin, 1-Ethylnaphthalin, Tetrahydronaphthalin, Diphenyl, Naphthalin; halogenierte aliphatische, cycloaliphatische oder aromatische Kohlenwasserstoffe wie Dichlordodecan, 1,5-Dibrompentan, Benzotrichlorid, o- und m-Dibrombenzol, Nitroverbindungen wie Nitrobenzol, 2-Nitrotoluol, Nitrile wie Benzylcyanid, Carbonylverbindungen wie Acetophenon oder das heterocyclische 2-Acetylthiophen, heterocyclische Verbindungen wie Chroman, Thiophhen, Ether wie Resorcindimethylether, Diphenylether, Safrol, Isosafrol, Säuren wie Önanthsäure, α-Ethylcapronsäure, Caprylsäure, Pelargonsäure, Versaticsäure, Caprinsäure, Ester wie Benzoesäureethylester, Phenylessigsäuremethylester und Salicylsäuremethylester. Solche geeigneten Lösungs- oder Verdünnungsmittel sind auch in der DE-AS 1 059 899 beschrieben, die als Referenz ausdrücklich einbezogen wird. Grundsätzlich können auch Lösungsmittelgemische verwendet werden.

Die folgenden Beispiele dienen zur Erläuterung der Erfindung.

Als Ausgangsmaterial dient ein polyesterhaltiges Umsetzungsprodukt, das analog der DE-AS 1 042 573, Beispiel 1, erhalten wird. Dabei werden in ein gerührtes Gemisch aus 800 g Crotonaldehyd, 1200 ml Toluol und 14,2 g Zinkisovalerianat bei einer Temperatur zwischen 25 und 35 °C 420 g Keten eingeleitet. Der Überschuss an Crotonaldehyd und das Toluol werden im Vakuum entfernt. Als Rückstand werden 1150 g Polyester (Sorbinsäurepolyester) in Form einer hochviskosen, braungefärbten Flüssigkeit erhalten. Neben dem Zinkgehalt von 3000 ppm enthält dieses Reaktionsprodukt noch Anteile, die nicht in Hexadiensäuren (Sorbinsäure) umgewandelt werden können, wie Diketenpolymerisate und Crotonaldehydharze.

Der in Hexadiensäuren umwandelbare Anteil wird bestimmt durch basische Verseifung einer Lösung aus 60 g Sorbinsäurepolyester in 120 g Toluol mit 33 g Kaliumhydroxid in 260 g Wasser bei Raumtemperatur. Dabei erhält man in der wässrigen Phase Kaliumsorbat und das Kaliumsalz der 3-Hydroxy-4-hexensäure, aus denen Hexadiensäuren durch Ansäuern gewonnen werden können. Durch quantitative Bestimmung der beiden Reaktionsprodukte mittels HPLC kann der in Hexadiensäuren umwandelbare Anteil des Polyesters bestimmt werden.

Durch diese milden Bedingungen lässt sich der Polyestergehalt genauer als in der DE-AS 1 282 645 beschrieben, bestimmen. So beträgt der zu Hexadiensäuren umwandelbare Anteil des Rohpolyesters 89 bis 90 % und nicht, wie in DE-AS 1 282 645 angenommen, nur 80 %. Die in der DE-AS 1 282 645 erzielten Ausbeuten müssen daher, siehe Beispiel 1 (Vergleichsbeispiel), entsprechend korrigiert werden.

### Beispiel 1 (Vergleichsbeispiel)

Die Apparatur besteht aus einem 11 - 3H-Rundkolben (Reaktionskolben) mit aufgesetzter Destillationskolonne. Die Destillationskolonne mit einer Füllhöhe von 600 mm und einem Innendurchmesser von 40 mm ist mit Raschigringen aus Glas von 6 mm Durchmesser gefüllt. Die Destillationskolonne trägt einen auf 70 °C gekühlten Kolonnenkopf mit Rücklaufteiler. Das kondensierte Destillat wird durch den Rücklaufteiler zum einen in die Kolonne zurückgeführt, zum anderen in einer graduierten Vorlage (500 ml) und einem 6 I Rundkolben aufgefangen. Die gesamte Apparatur wird unter Vakuum betrieben; zur Vakuumerzeugung dient eine Ölpumpe mit vorgeschalteter Trockeneiskühlfalle.

Zum Einsatz kommen jeweils 260 g Destillationsrückstand aus einem Vorversuch, der 40 % Dimethylstearylamin (DMSA) enthält. Die Apparatur wird auf etwa 30 mbar evakuiert und der Reaktionskolben mit dem Ölbad (Badtemperatur ca 220 °C) angeheizt. Erreicht die Temperatur im Reaktionskolben 180 °C wird mit der Dosierung (417 g/h) des Einsatzgemisches in den Reaktionskolben begonnen. Sobald Destillat anfällt, wird ein Rücklauf auf 417 g/h eingestellt.

Das Einsatzgemisch in den Reaktionskolben besteht aus 350 g Polyester, 2128 g 2-Ethylhexansäure, 48 g DMSA und 14 g Arcopal® (Gesamtmenge 2540 g). Nach der Dosierung des Einsatzgemisches wird zum Nachspalten von noch nicht umgesetztem Polyester für 2 h reines Lösungsmittel (Nonylphenolpolyglycolether) ohne Polyester und ohne Rücklauf durch die Apparatur gefahren (834 g) und danach 5 min nachdestilliert. Das in der Vorlage befindliche Destillat wird durch Erwärmen auf 50 bis 55 °C homogenisiert und anschließend unter Rühren (500 Umin ⁻¹) innerhalb von 3 h auf 20 °C abgekühlt. Nach Erreichen dieser Temperatur wird es noch 15 min bei 20 °C gehalten und danach die Rohsorbinsäure abgesaugt und der Reingehalt gaschromatographisch bestimmt. Die Mutterlauge wird im nächsten Versuch wieder eingesetzt.

Man erhält eine Ausbeute an Sorbinsäure von 82,1 %. Bezogen auf den Reinpolyester, also unter alleiniger Berücksichtigung des zu Hexadiensäuren spaltbaren Anteils von 90 %, errechnet sich damit eine Ausbeute von 91, 2 %.

### Beispiel 2

Die Apparatur besteht aus einem 1l - 3H-Rundkolben (Reaktionskolben) mit aufgesetzter Destillationskolonne. Die Destillationskolonne mit einer Füllhöhe von 600 mm und einem Innendurchmesser von 40 mm ist mit Raschigringen aus Glas von 6 mm Durchmesser gefüllt. Die Destillationskolonne trägt am Kolonnenkopf einen auf 100 °C beheizten Einlauf und eine isolierte Claisenbrücke mit absteigendem, 70 °C warmen Kühler. An den Kühler sind nacheinander eine graduierte, beheizbare Vorlage (500 ml) und ein 6 I Rundkolben angeschlossen. Die gesamte Apparatur wird unter Vakuum betrieben; zur Vakuumerzeugung dient eine Ölpumpe mit vorgeschalteter Trockeneiskühlfalle. Zum Einsatz kommen jeweils 260 g Destillationsrückstand aus einem Vorversuch, der 40 % Dimethylstearylamin enthält. Die Apparatur wird auf etwa 30 mbar evakuiert und der Reaktionskolben mit dem Ölbad (Badtemperatur ca. 220 °C) angeheizt. Erreicht die Temperatur im Reaktionskolben 180 °C wird mit der Dosierung (417 g/h) des Einsatzgemisches in den Reaktionskolben begonnen. Sobald Destillat anfällt, wird 2-Ethylhexansäure (ebenfalls 417 g/h, beim ersten Versuch Lösungsmittel aus dem Betrieb) über einen Wärmetauscher und den beheizbaren Einlauf (beide auf 100 °C erwärmt) am Kopf der Kolonne eindosiert.

Das Einsatzgemisch in den Reaktionskolben besteht aus 350 g Polyester, 2128 g 2-Ethylhexansäure, 48 g DMSA und 14 g Arcopal® (Gesamtmenge 2540 g). Gleichzeitig wird die gleiche Menge (2540 g) 2-Ethylhexansäure als Rücklauf dosiert.

Die Reaktionsprodukte und das Lösungsmittel werden mit möglichst geringem wildem Rücklauf abdestilliert. Nach der Dosierung des Einsatzgemisches und des Lösungsmittelrücklaufs wird für 2 h reines Lösungsmittel (2-Ethylhexansäure) ohne Polyester und ohne Rücklauf durch die Apparatur gefahren (834 g) und danach 5 min nachdestilliert. Das in der Vorlage befindliche Destillat wird durch Erwärmen auf 50 bis 55 °C homogenisiert und anschließend unter Rühren (500 Umin ⁻¹) innerhalb von 3 h auf 20 °C abgekühlt. Nach Erreichen dieser Temperatur wird noch 15 min bei 20 °C gehalten und danach die Rohsorbinsäure abgesaugt und der Reingehalt gaschromatographisch bestimmt. Die Mutterlauge wird im nächsten Versuch wieder zum Ansetzen des Einsatzgemisches und des Rücklaufs eingesetzt. Man erhält eine Ausbeute an Sorbinsäure von 84,2 %. Bezogen auf den Reinpolyester, also unter alleiniger Berücksichtigung des zu Hexadiensäuren spaltbaren Anteils von 90 %, errechnet sich damit eine Ausbeute von 93,6 %.

### Beispiel 3

Aufbau und Fahrweise entspricht der in Beispiel 2. Im Einsatzgemisch wird die Sorbinsäurepolyestermenge auf 400 g erhöht und die Menge an Lösungsmittel auf 2078 g erniedrigt. Die Menge an Dimethylstearylamin und Arcopal und damit der Gesamtflüssigkeitseinsatz pro Zeit bleiben gleich. Man erhält eine Ausbeute an Sorbinsäure von 83,7 %. Bezogen auf den Reinpolyester, also unter alleiniger Berücksichtigung des zu Hexadiensäuren spaltbaren Anteils von 90 %, errechnet sich damit eine Ausbeute von 93 %.

### Beispiel 4

Aufbau und Fahrweise entspricht der in Beispiel 2. Im Einsatzgemisch wird die Sorbinsäurepolyestermenge auf 450 g erhöht und die Menge an Lösungsmittel auf 2028 g erniedrigt. Die Menge an Dimethylstearylamin und Arcopal und damit der Gesamtflüssigkeitseinsatz pro Zeit bleiben gleich. Man erhält eine Ausbeute an Sorbinsäure von 82,9%. Bezogen auf den Reinpolyester, also unter alleiniger Berücksichtigung des zu Hexadiensäuren spaltbaren Anteils von 90 %, errechnet sich damit eine Ausbeute von 92,1 %.

### Beispiel 5

Aufbau und Fahrweise entspricht der in Beispiel 2. Im Einsatzgemisch wird die Sorbinsäurepolyestermenge auf 500 g erhöht und die Menge an Lösungsmittel auf 1978 g erniedrigt. Die Menge an Dimethylstearylamin und Arcopal und damit der Gesamtflüssigkeitseinsatz pro Zeit bleiben gleich. Man erhält eine Ausbeute an Sorbinsäure von 80,6 %. Bezogen auf den Reinpolyester, also unter alleiniger Berücksichtigung des zu Hexadiensäuren spaltbaren Anteils von 90 %, errechnet sich damit eine Ausbeute von 89,6 %.

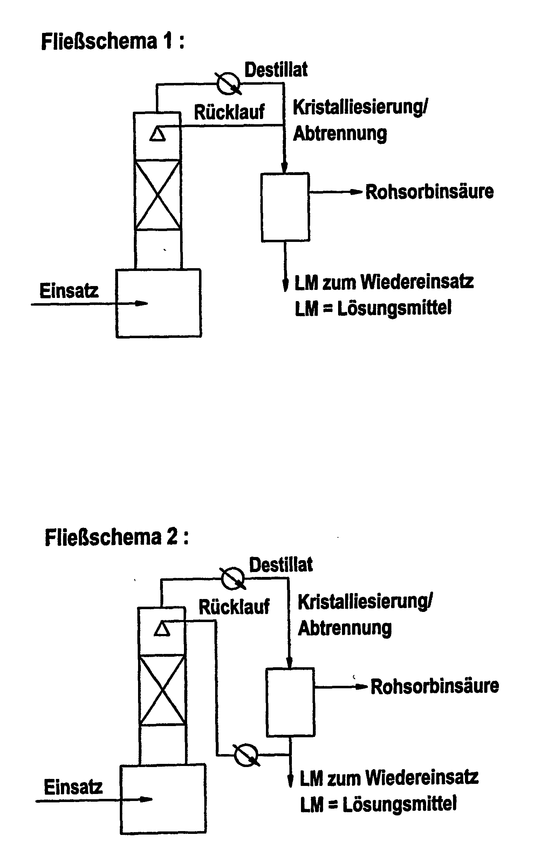

## Patentansprüche

1. Verfahren zur Herstellung von Sorbinsäure durch thermische Spaltung des aus Crotonaldehyd und Keten hergestellten Polyesters in Gegenwart eines Lösungsmittels und eines Amins als Katalysator und unter gleichzeitigem Abdestillieren der gebildeten Sorbinsäure und des Lösungsmittels über eine Rektifikationskolonne mit Rücklauf, **dadurch gekennzeichnet, dass** als Rücklauf das Lösungsmittel verwendet wird.

2. Verfahren nach Anspruch 1, **dadurch gekennzeichnet, dass** als Lösungsmittel solche Substanzen verwendet werden, die mit der Sorbinsäure azeotrope Gemische bilden.

3. Verfahren nach Anspruch 1 oder 2, **dadurch gekennzeichnet, dass** der Siedepunkt des Lösungsmittels oberhalb von 150 °C, bevorzugt oberhalb von 180 °C, und unterhalb von 300 °C liegt.

4. Verfahren nach einem oder mehreren der Ansprüche 1 bis 3, **dadurch gekennzeichnet, dass** aliphatische, alicyclische, aromatische Kohlenwasserstoffe, deren Chlor-, Brom- und Nitroderivate sowie Äther, Siliconöle, Ketone, Ester, Carbonsäuren und Alkohole, deren Siedepunkte bei normalem Druck über 150 °C liegen, als Schleppmittel oder Lösungsmittel verwendet werden.

5. Verfahren nach einem oder mehreren der Ansprüche 1 bis 4, **dadurch gekennzeichnet, dass** sekundäre oder tertiäre aliphatische, alicyclische, 5- oder 6-gliedrige heterocyclische Stickstoff und/oder Sauerstoff enthaltende oder aliphatisch-aromatisch substituierte, unter Normaldruck oberhalb 100 °C siedende Amine als Katalysator verwendet werden.

6. Verfahren nach einem oder mehreren der Ansprüche 1 bis 5, **dadurch gekennzeichnet, dass** die thermische Spaltung des Polyesters bei 160 - 220 °C durchgeführt wird.
